# FASCICULE DE BREVET EUROPEEN

(11) **EP 2 164 532 B1**
(45) Date de publication et mention de la délivrance du brevet: **01.02.2012**
(21) Numéro de dépôt: 08826127.6
(22) Date de dépôt: 10.06.2008
(51) Int. Cl.: A61L 15/32, A61L 15/28, A61L 15/44, A61K 38/42, A61L 15/22, A61K 8/64, A61Q 19/00, A61P 1/04

(54) **UTILISATION D'UNE HEMOGLOBINE POUR LA PREPARATION DE PANSEMENTS, ET PANSEMENTS AINSI PREPARES**
VERWENDUNG EINES HÄMOGLOBINS ZUR HERSTELLUNG VON VERBÄNDEN UND DARAUS RESULTIERENDE VERBÄNDE
USE OF A HAEMOGLOBIN FOR THE PREPARATION OF DRESSINGS AND RESULTING DRESSINGS

(30) Priorité: 18.06.2007 FR 0704312
(43) Date de publication de la demande: 24.03.2010
(73) Titulaire: Centre National de la Recherche Scientifique, 75794 Paris Cedex 16 (FR); Hemarina SA, 29679 Morlaix Cedex (FR); Université Pierre et Marie Curie, 75252 Paris Cedex 05 (FR)
(72) Inventeur: ZAL, Franck, F-29600 Morlaix-Ploujean (FR); ROUSSELOT, Morgane, F-29250 Saint-Pol de Leon (FR)
(74) Mandataire: Cabinet Plasseraud
(86) Numéro de dépôt international: PCT/FR2008/000786
(87) Numéro de publication internationale: WO 2009/007532

(56) Documents cités:
- US-A- 4 959 341
- US-A1- 2003 180 365
- US-A1- 2004 022 839
- US-A1- 2004 086 493
- US-A1- 2006 134 186

## Description

La présente invention concerne l'utilisation d'une hémoglobine pour la préparation de pansements, et les pansements ainsi préparés.

L'hémoglobine présente dans le flux sanguin humain ou animal transporte l'oxygène des poumons jusqu'aux extrémités des membres. L'oxygène possède une très faible solubilité en milieu aqueux, et est donc distribué dans l'organisme, très près des cellules, par l'hémoglobine grâce au réseau de capillaires, par diffusion.

Lorsqu'il existe une blessure ouverte au niveau de la peau, l'approvisionnement de l'oxygène par diffusion à la surface des cellules est alors supprimé.

La peau est un tissu complexe assurant des fonctions élémentaires de protection, de sensibilité, de thermorégulation et de métabolisme. L'interruption dans la continuité des tissus (plaie) peut retentir sur chacune de ces fonctions.

Deux types de plaies peuvent être distingués selon le délai de cicatrisation : les plaies aiguës et les plaies chroniques. La cicatrisation des premières se fait sans complication et en moins de six semaines. Les secondes se définissent comme des lésions cutanées dont le délai de cicatrisation est supérieur à six semaines.

Les plaies chroniques regroupent entre autres les escarres, les ulcères de jambe et les ulcères du « pied diabétique ».

L'escarre est une lésion cutanée d'origine ischémique liée à une compression des tissus mous entre un plan dur et les saillis osseuses.

L'ulcère de jambe est une plaie située sous le genou n'ayant pas cicatrisée spontanément, en général d'origine veineuse.

Le « pied diabétique » est une conséquence de complications vasculaires et neurologiques du diabète au niveau des pieds. Ces plaies sont toutes liées directement où indirectement, à un problème d'oxygénation.

Par exemple, dans le cas des escarres, la compression entraîne une réduction de l'apport sanguin et donc de l'apport en O₂ (hypoxie) et en nutriments au niveau cellulaire, ce qui conduit à l'ischémie, puis à la nécrose. La compression engendre une obstruction veineuse et donc une stase suivie d'une obstruction des capillaires puis des artères musculo-cutanées.

L'ischémie (diminution de la circulation sanguine) résulte essentiellement de l'atteinte du système vasculaire. Cela conduit à la réduction des quantités d'O₂ et de nutriments à l'origine du retard de cicatrisation des plaies. L'infection peut-être superficielle mais son risque est lié à une atteinte profonde pouvant menacer les tissus, les gaines et tendons, et surtout les structures osseuses.

Les maladies parodontales sont également très fréquentes chez les patients diabétiques. Très souvent, le système immunitaire de ces patients ne permet pas d'une façon efficace de faire face à une infection de type bactérien. Au niveau buccal, il s'en suit un déséquilibre de la flore buccale avec une prolifération de pathogènes anaérobies.

De nombreux facteurs externes sont également responsables de cette pathologie qui dans la plupart du temps est due à une prolifération de bactéries anaérobies.

Pour promouvoir la cicatrisation et notamment celle des plaies chroniques ouvertes, plusieurs techniques ont été développées ces dernières années, et notamment l'utilisation d'hémoglobine seule ou en association avec un gel.

Ainsi, le brevet EP 0 862 440 décrit l'utilisation thérapeutique de l'hémoglobine de tout type pour favoriser la cicatrisation chez un patient. L'hémoglobine est administrée par voie intraveineuse et peut être naturelle ou modifiée chimiquement. Cependant, l'hémoglobine naturelle présente deux problèmes à son utilisation: d'une part son instabilité dans le temps et d'autre part la possibilité de déclenchement d'une réaction allergique chez le patient.

Le brevet US 4,959,341 décrit des gels biodégradables detenus par mélange entre un acide carbosylique hydrophobe, un hydrate de carbon et un agent réticulant.

La demande de brevet US 2003/0180365 concerne une préparation applicable par voie externe, contenant un transporteur d'oxygène, dans laquelle le transporteur d'oxygène est incorporé, et dispersé moléculairement, dans une préparation ayant une consistance de gel, pour la régénération de la peau en cas de déficience d'oxygène.

Le gel utilisé peut être un gel inorganique, ou un gel organique. Le transporteur d'oxygène peut être de l'hémoglobine native humaine ou animale ou un mélange de ladite hémoglobine avec une myoglobine de cheval, chien ou mouton.

Ce gel libère l'hémoglobine transportant l'oxygène au niveau de la peau pour permettre une diffusion de l'oxygène dans l'épiderme. Ce gel ne possède pas de propriétés bactéricides et n'est donc pas utilisable dans le cadre de plaies ouvertes présentant un exsudat trop important, car il y a alors nécessité de lutter contre l'infection présente dans l'exsudat.

Préférentiellement, l'hémoglobine est protégée de l'oxydation, c'est-à-dire stabilisée, notamment avec CO. Il est précisé dans ce document que l'hémoglobine peut être utilisée sans stabilisation, mais qu'une telle préparation ne peut être conservée aussi longtemps que la préparation stabilisée, à cause de l'oxydation de l'hémoglobine.

La demande US2004/0022839 décrit une formulation applicable par voie externe, contenant un transporteur d'oxygène, dans laquelle le transporteur d'oxygène est incorporé dans une émulsion de lipoïdes.

Cette formulation est destinée à la régénération de la peau en cas de déficience d'oxygène et n'est pas utilisable non plus pour le traitement de plaies ouvertes.

Le transporteur d'oxygène peut être de l'hémoglobine et la formulation libère l'hémoglobine transportant l'oxygène au niveau de la peau pour permettre une diffusion de l'oxygène dans l'épiderme.

Les mêmes problèmes que précédemment sont rencontrés avec cette formulation, à savoir l'oxydation de l'hémoglobine, si celle-ci n'est pas stabilisée avec un antioxydant et la nécessité d'utiliser des cofacteurs naturels tel que du 2,3-diphosphoglycérate, ou non naturels tels que l'inositol hexaphosphate ou l'acide mellitique, pour obtenir une action sur la régénération de l'épiderme.

La demande US2005/0129747 décrit l'utilisation d'un transporteur d'oxygène, modifié ou non pour la production d'un agent pour le traitement externe des plaies ouvertes.

Le transporteur d'oxygène peut être une hémoglobine ou une myoglobine d'origine humaine ou animale, modifiée ou non, et le transporteur est utilisé en solution ou en pulvérisation.

L'hémoglobine ainsi déposée dans la barrière aqueuse de la plaie ouverte permet la diffusion de l'oxygène à travers cette barrière pour favoriser la cicatrisation.

Les limitations de ce transporteur résident dans l'oxydation de l'hémoglobine native et par conséquent, la nécessité d'utilisation d'antioxydants, de cofacteurs ou d'hémoglobine modifiée chimiquement.

La demande US 2005/0232953 décrit des micro-émulsions d'eau dans l'huile comprenant une hémoglobine, modifiée ou non ainsi que des antioxydants.

Il est précisé dans cette demande que les cellules vitales de l'épithélium de la peau sont protégées de l'environnement extérieur par le stratum coméum, qui est difficile à pénétrer. Par conséquent, les deux conditions, selon cette demande, pour qu'une substance active contourne la barrière du stratum cornéum sont les suivantes :
1) pénétration : entrée de la substance dans le stratum cornéum,
2) perméation : diffusion de la substance du stratum cornéum vers l'épiderme.

Selon cette demande, l'hémoglobine contenue dans la microémulsion, pénètre rapidement et profondément dans le stratum cornéum et y diffuse l'oxygène.

Cependant, cette hémoglobine va rapidement s'oxyder dans le stratum cornéum et nécessite par conséquent la présence d'un anti-oxydant car il est bien connu que lorsque l'on isole une hémoglobine du globule rouge, celle-ci va s'oxyder du fait de l'absence de l'activité anti-oxydante des enzymes présentes dans le globule rouge. (Savitsky JP, Doczi J, Black j & Arnold JD (1978) A clinical safety trial of stroma-free hemoglobin. Clin Pharmacol Ther 23, 73-80), (Chan WL, Tang NL, Yim CC, Lai FM & Tam MS (2000); New features of renal lesion induced by stroma free haemoglobin, Toxicol Pathol 28, 635-642).

Par conséquent, l'un des objets de l'invention est de fournir un pansement contenant de l'hémoglobine immobilisée et stable dans une matrice permettant le traitement des plaies ischémiques.

Un autre objet de l'invention est de fournir un pansement à base d'hémoglobine ne nécessitant pas l'utilisation de cofacteurs ou d'anti-oxydants.

Un autre aspect de l'invention est de fournir un pansement à base d'hémoglobine permettant une utilisation prolongée dans le temps et évitant le déclenchement de réactions allergiques.

Un autre objet de l'invention est de fournir des compositions cosmétiques ou pharmaceutiques comprenant de l'hémoglobine dans une matrice pour le traitement des plaies ischémiques et/ou d'infections locales causées par des pathogènes, notamment anaérobies.

Un autre objet de l'invention est de fournir un procédé de préparation de l'hémoglobine immobilisée dans une matrice.

Par conséquent, l'invention concerne l'utilisation d'une hémoglobine, humaine ou d'animal vertébré ou invertébré, essentiellement immobilisée dans une matrice et stable dans la matrice, ladite matrice étant physiologiquement acceptable et à base d'hydrocolloïdes avantageusement polymérisés, et contenant de 0 à 98% d'humidité, comme transporteur d'oxygène dans un tissu physiologique, notamment hypoxique, nécessitant un apport d'oxygène, sans que le relargage de l'hémoglobine essentiellement immobilisée dans la susdite matrice ne soit supérieur à 10% en poids.

Par hémoglobine essentiellement immobilisée dans une matrice, il faut comprendre que l'hémoglobine reste dans la matrice et n'est presque pas relarguée, c'est-à-dire libérée à l'extérieur de celle-ci.

La proportion d'hémoglobine relarguée est mesurée dans des conditions in vivo simulées par dosage colorimétrique au réactif de Drabkin's comme explicité dans l'exemple 5.

L'expression « stable dans la matrice » signifie que l'hémoglobine n'est pas oxydée dans la matrice et qu'elle ne se dénature pas.

En particulier le pansement de l'invention ne se liquéfie pas au contact de la plaie, évitant ainsi de libérer l'hémoglobine.

La matrice représente le milieu dans lequel l'hémoglobine est immobilisée et est constituée d'hydrocolloïdes, c'est-à-dire un agent de fixation d'origine végétale.

Donc, l'hémoglobine ainsi piégée dans la matrice ne pénètre pas dans la peau, que ce soit dans le stratum cornéum ou dans l'épiderme ce qui va permettre ainsi la diffusion permanente de l'oxygène dans le stratum cornéum.

Par animal vertébré, on désigne un bovin, un mammifère tel que le porc, un mouton, un singe ou un serpent, et par animal invertébré, on désigne un insecte, ou appartient à l'embranchement des annélides.

Par tissu physiologique « hypoxique », on désigne un tissu pauvre ou amoindri en oxygène (par exemple, lorsque le tissu désigne une plaie, la pression partielle transcutanée en O₂ est inférieure à 40mmHg ; Smith B, Devigne L, Slade J, Dooley J & Warren D. Wound Rep Reg. 4, 224 (1996)).

L'hémoglobine immobilisée dans une matrice constitue un pansement, terme qui pourra être utilisée par la suite pour désigner ladite hémoglobine immobilisée dans une matrice.

La matrice contient de 0 à 98% d'eau car elle peut être sous forme sèche, c'est-à-dire contenir de 0 à 5% d'eau, ou sous forme humide, c'est-à-dire contenir de 50 à 98% d'eau.

Dans un mode de réalisation préféré, l'invention concerne l'utilisation d'une hémoglobine immobilisée dans une matrice, telle que définie ci-dessus, pour la préparation d'un pansement destiné au traitement externe de plaies ouvertes, profondes ou chroniques, ou de maladies parodontales, ou pour la préparation d'une, composition pharmaceutique, destinée à un pansement gastrique ou pour la préparation de composition cosmétiques.

Les plaies ouvertes sont des lésions provoquées par un agent extérieur et qui entraînent une ouverture cutanée.

Les plaies profondes sont des plaies dans lesquelles les muscles puis les os ou les organes peuvent être atteints.

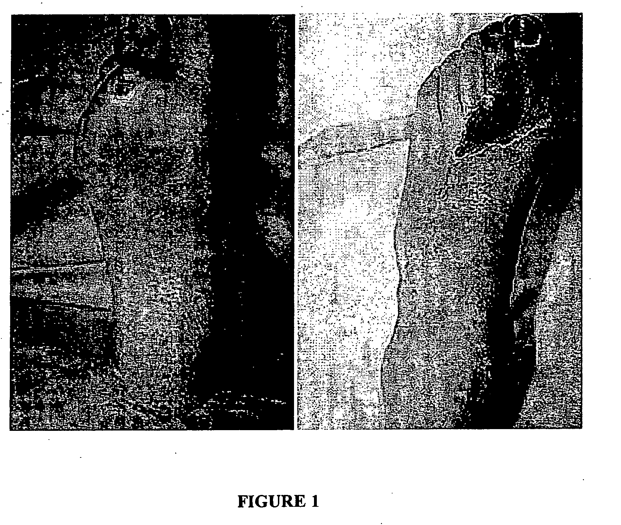

Les plaies chroniques regroupent notamment les escarres, les ulcères de jambe et les ulcères du pied diabétique (figure 1).

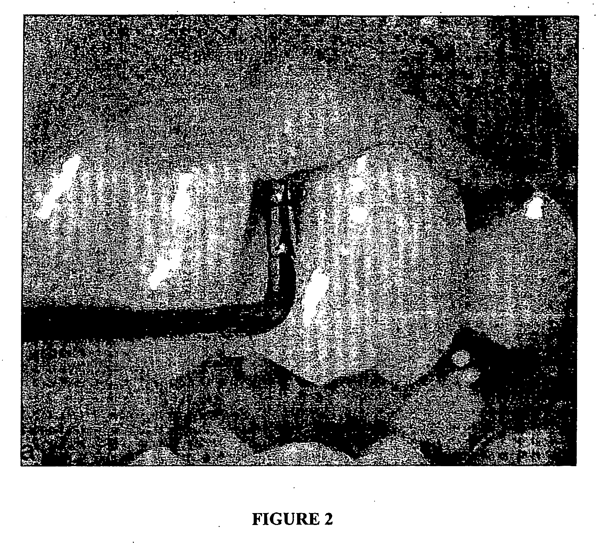

Les maladies parodontales concernent tous les tissus de soutien des dents : la gencive (figure 2), le ligament et l'os alvéolaire.

Par « pansement gastrique », il faut comprendre un pansement capable de former une pellicule protégeant la muqueuse gastrique et permettant d'agir ainsi en tant que protecteur gastrique pour le traitement notamment des gastrites, des brûlures oesophagiennes ou du météorisme.

Les compositions cosmétiques sont destinées à la régénération de la peau dans le cas de déficience en oxygène ou à la prévention de cette déficience et notamment dans le cadre des modifications dégénératives de la peau, dé modifications induites par les radiations, par la chaleur ou par l'âge, et particulièrement les rides.

L'invention concerne l'utilisation d'une hémoglobine immobilisée dans une matrice telle que définie ci-dessus, dans laquelle la matrice est formée d'un réseau tridimensionnel délimitant des pores dont la taille est comprise d'environ 2nm à environ 300µm, préférentiellement d'environ 2nm à environ 10µm, préférentiellement d'environ 2nm à environ 1µm, encore plus préférentiellement d'environ 5nm à environ 200nm et préférentiellement d'environ 15nm.

La taille des pores est avantageusement de 15 nm pour pouvoir contenir l'hémoglobine.

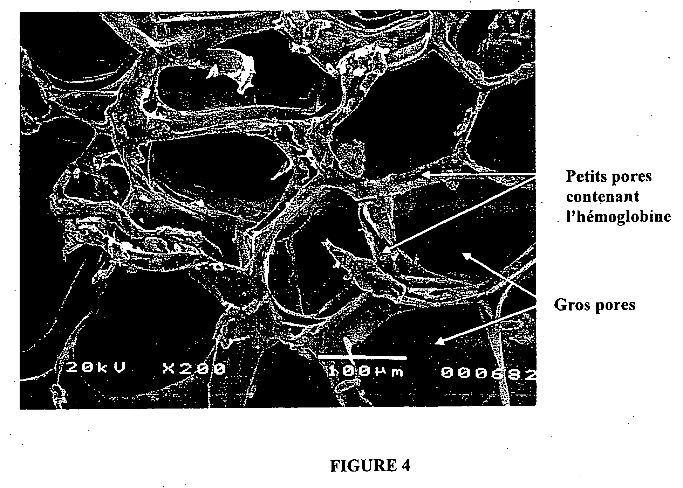

Les pores sont représentés dans la figure 4. Il existe deux types de pores dans la matrice, des gros pores mesurant environ 150µm et des pores beaucoup plus petits, de l'ordre de la dizaine de nanomètres, et contenant l'hémoglobine. Ces pores sont situés dans les membranes entourant les gros pores et contiennent l'hémoglobine.

Dans un mode de réalisation préféré, dans l'utilisation de l'hémoglobine stabilisée dans la matrice , la quantité d'hémoglobine par rapport au poids sec total d'hémoglobine et de matrice est comprise d'environ 0.1% (p/p) à environ 60% (p/p), préférentiellement d'environ 10% (p/p) à environ 50% (p/p), préférentiellement d'environ 15% (p/p) à environ 45% (p/p), préférentiellement d'environ 30% (p/p) à environ 40% (p/p) et plus préférentiellement environ 40% (p/p).

La quantité d'hémoglobine est ici indiquée en poids sec.

Si la quantité d'Hémoglobine est inférieure à 0.1%, le transport de l'oxygène ne sera plus efficace.

Si la quantité d'hémoglobine est supérieure à 60%, le pansement relargue alors trop d'hémoglobine.

Une quantité d'hémoglobine de 40% permet d'avoir le meilleur compromis entre l'efficacité de transport de l'oxygène et un moindre relargage.

Selon un mode de réalisation avantageux; l'invention concerne l'utilisation d'une hémoglobine immobilisée dans une matrice, telle que définie ci-dessus, dans laquelle le pourcentage d'humidité est compris de 0% à environ 98%, et préférentiellement environ 50%.

Un pourcentage d'humidité de 50% représente le meilleur compromis entre le faible relargage, l'efficacité de transport d'oxygène et la facilité de manipulation du pansement.

Selon un mode de réalisation préféré de l'invention, l'hémoglobine est de l'hémoglobine humaine ou d'animal vertébré, modifiée chimiquement, ou réticulée.

L'hémoglobine d'animal vertébré peut être une hémoglobine de bovin, de mammifère tel que le porc, un mouton, un singe ou un serpent.

L'hémoglobine peut être modifiée chimiquement par exemple avec du CO pour obtenir une carboxyhémoglobine.

L'hémoglobine peut être réticulée en créant un pont chimique entre deux de ses quatre chaînes polypeptidiques et en liant plusieurs molécules d'hémoglobines entre elles selon les techniques connues dans la littérature (J. M. Harris (éditeur) : Poly-ethylene glycol chemistry :Biotechnical and Biomedical Application, Plenum, New York et al. 1992).

Les agents de réticulation utilisés peuvent être des polypropylènes glycols ou des polyéthylènes glycols ou des dialdéhydes.

Dans un mode de réalisation préféré de l'invention, l'hémoglobine est une hémoglobine extracellulaire d'animal invertébré, choisie parmi le phylum des annélides et est notamment une hémoglobine extracellulaire appartenant à des vers marins tels *qu'Arenicola marina.*

On distingue dans le phylum des annélides, 3 classes ; les Polychètes (tel que *Arenicola marina*), les Oligochètes (tel que le vers de terre *Lumbricus terrestris*) et les Achètes (tel que les sangsues).

Les annélides sont des animaux protostomiens segmentés (possédant des métamères quelquefois très nombreux) et en forme de « ver ». Ils vivent essentiellement dans l'eau (eau de mer comme la néréis ou eau douce comme la sangsue) même si certaines espèces comme les lombrics vivent dans le sol.

L'utilisation d'une hémoglobine extracellulaire, stable dans la matrice permet d'éviter l'utilisation d'anti-oxydant et/ou de cofacteur pour fonctionner.

L'utilisation d'une hémoglobine extracellulaire, stable dans la matrice permet de mettre en jeu l'activité SOD intrinsèque (déterminée par la méthode de Flohé & Ötting; Flohé L, Otting F. Methods Enzymol (1984), 105, 93-104) de ladite hémoglobine, lui procurant ainsi une activité anti-oxydante intrinsèque, et ne nécessitant, par conséquent, aucun anti-oxydant ou cofacteur pour fonctionner.

Selon un mode de réalisation avantageux de l'invention ladite matrice est à base de chitosane, de carraghénanes, de carboxyméthylcellulose, d'alginates et en particulier d'alginate de sodium ou de calcium.

L'alginate de calcium peut être obtenue par échange d'ion de l'alginate de sodium, par réaction de l'alginate de sodium avec un cation divalent tel que le chlorure de calcium, l'acétate de calcium, le carbonate de calcium, le phosphate de calcium, de préférence le chlorure de calcium.

Le chitosane est produit par désacétylation de la chitine présent dans l'exosquelette des insectes et autres arthropodes (crustacés, arachnides, etc.).

C'est un polysaccharide aminé constitué de groupes N-acétyl-D-glucose-2-amine reliés entre eux par une liaison du type β-(1,4).

Les carraghénanes sont des polysaccharides linéaires constitués de molécules de galactoses plus ou moins substitués. La chaîne est constituée de sous unités appelées carrabioses comprenant deux galactoses liés par une liaison β-(1,4). Ces carrabioses sont liés entre eux dans la chaîne par des liaisons α-(1,3).

La carboxyméthylcellulose est un polymère dérivé de la cellulose naturelle formé par réaction de la cellulose avec une base et de l'acide chloroacétique. Il est basé sur une structure β-(1,4)-D-glucopyranose.

Les alginates sont des polymères d'acide alginique (constitué d'acide mannuronique et d'acide guluronique) obtenus à partir d'algues brunes (Laminariales, Fucales). L'alginate de sodium est extrait des algues avec de la soude puis séché pour obtenir une poudre blanche, d'un poids moléculaire allant de 32 000 à 200 000 Da.

Dans un mode de réalisation préféré de l'invention, le rapport dudit alginate sur le poids total d'hémoglobine et de matrice est d'environ 40%(p/p) à environ 99%(p/p), préférentiellement d'environ 50%(p/p) à environ 90%(p/p), préférentiellement d'environ 55%(p/p) à environ 85%(p/p), préférentiellement d'environ 60%(p/p) à environ 80%(p/p), et préférentiellement environ 60%(p/p).

La quantité d'alginate utilisé ici est indiquée en poids sec.

Si le rapport est inférieur à 40%, on observe alors un relargage de l'hémoglobine.

Si le rapport est supérieur à 60%, l'efficacité de transport de l'oxygène est diminuée.

Selon un mode de réalisation avantageux, l'invention concerne l'utilisation d'une hémoglobine immobilisée dans une matrice, telle que définie ci-dessus, qui permet la création d'un gradient d'oxygène au travers du gel, vers la zone déficiente en oxygène.

L'un des avantages de l'invention réside dans l'immobilisation de l'hémoglobine dans la matrice avec moins de 10% de relargage.

Cette immobilisation permet la fixation de l'oxygène de l'air par l'hémoglobine à travers le gel puis la libération de l'oxygène au niveau de la plaie, aboutissant ainsi à la création d'un gradient d'oxygène.

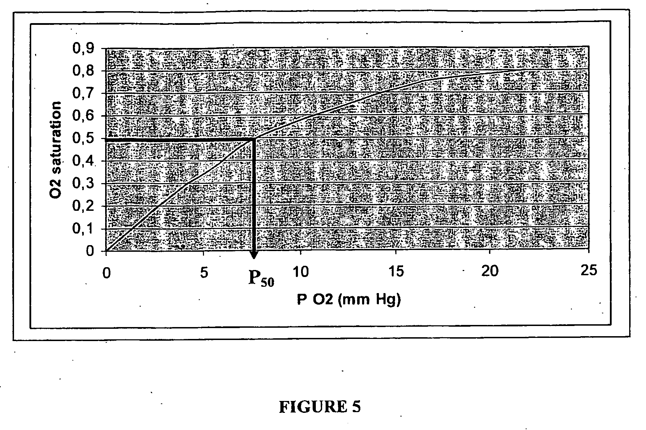

A titre d'exemple, l'affinité de l'hémoglobine *d'Arenicola marina* dans le pansement est de P₅₀=7-8 (la P₅₀ est la PO₂ pour laquelle 50% de l'hémoglobine *d'Arenicola marina* est saturée en O₂), c'est-à-dire que la matrice fixe l'oxygène de l'air si la PO2 est supérieure à 8 et le libère si la PO₂ est inférieure à 7 (Figure 5)

Dans un mode de réalisation préféré de l'invention, l'hémoglobine est en association avec un ou plusieurs éléments choisis parmi des cofacteurs, des conservateurs tels que le 4-hydroxybenzoate de méthyle, le 4-hydroxybenzoate de propyle, des antioxydants tels que le gluthation réduit, l'acide ascorbique, le NADH, de la myoglobine humaine ou d'origine animale, ou une combinaison de ces éléments.

Bien que l'hémoglobine de l'invention puisse fonctionner sans anti-oxydants ni cofacteurs, il peut être avantageux, si nécessaire, pour accroître encore la durée de vie du pansement, de rajouter des anti-oxydants ou des conservateurs, ou des cofacteurs et/ou de la myoglobine qui permettent d'optimiser le fonctionnement du pansement.

Selon un mode de réalisation avantageux, l'utilisation d'une hémoglobine immobilisée dans une matrice, selon l'invention, permet l'exercice d'un effet bactéricide sur les bactéries anaérobies de type Gram

Dans le cadre des maladies parodontales (figure 2), ce sont les bactéries anaérobies, les plus pathogènes, qui sont responsables de la pathologie (Tableau 1).

Ces pathogènes très réfractaires aux traitements antibiotiques provoquent des poches entre la gencive et la dent qui peut entraîner des lésions osseuses et la perte de l'organe dentaire.

**Tableau 1 :Liste des pathogènes responsables de maladies parodontales. La plupart de ces bactéries sont des micro-organismes anaérobies.**

| | GC | PPP | PJL | PJG | PPR | PA | PAA | PAT | PR | P-HIV | GUN |
|---|---|---|---|---|---|---|---|---|---|---|---|
| Actinobacillus | | | | | | | | | | | |
| actinomycetemcomitans | | + | +++ | | | + | ++ | | | + | |
| Eikenella corrodens | ++ | + | | | + | + | | | | + | |
| Capnocytopaga species | ++ | | | | + | + | | | | + | |
| Porphyromanas | | + | | ++ | +++ | + | | | | + | |
| gingivalis | | | | | | | | | | | |
| Prevotella intermedia | + | + | + | | | + | ++ | ++ | ++ | + | +++ |
| Bacteroides forsythus | | | | | | + | | | | + | |
| Prevotella | | | | | | + | | | | + | |
| melanogenica | | | | | | | | | | | |
| Fusobacterium | ++ | + | | | | + | | | ++ | + | + |
| nucleatum | | | | | | | | | | | |
| Campylobacter rectus | + | | | | | + | | | | +++ | |
| Peptostreptococcus | | | | | | | | | | | |
| micros | | | | | | + | | | | + | |
| Treponema species | | | | | + | + | ++ | | + | ++ | +++ |
| Entérobactéries | | | | | | | | | + | + | |
| Bactéries Gr+ aéro- | +++ | | | | | | | | | | |
| anaérobie | | | | | | | | | | | |

| | | | | | | | | | | | |
|---|---|---|---|---|---|---|---|---|---|---|---|
| GC = gingivite chronique (ou gingivite associée à la plaque), PPP = parodontite pré-pubertaire, PJL = parodontite juvénile localisée (ou parodontite agressive), PJG = parodontite juvénile généralisée (ou parodontite agressive), PPR = parodontite à progression rapide (ou parodontite agressive), PA = parodontite de l'adulte (ou parodontite chronique localisée/généralisée). PAA = phase active de parodontite de l'adulte (ou parodontite agressive), PAT = parodontite associée au tabac (ou parodontite agressive), PR = parodontite réfractaire, P-HIV = parodontite associée au HIV (parodontite nécrosante), GUN = gingivite ulcéro-nécrotique (parodontite nécrosante). Fréquence d'isolement des bactéries pathogènes : +, ++, +++. | | | | | | | | | | | |

En effet, lorsqu'une plaie présente un exsudat; celui-ci contient des bactéries qui provoquent une infection et il y a alors nécessité de lutter à la fois contre l'exsudat et contre l'infection.

Le pansement de l'invention permet par conséquent de lutter contre l'infection en drainant l'exsudat, l'oxygène alors présent dans le pansement peut exercer son activité bactéricide, et le gradient d'oxygène créé favorise la cicatrisation.

Dans un autre aspect, l'invention concerne un pansement comprenant une hémoglobine, humaine ou d'animal vertébré ou invertébré, essentiellement immobilisée dans une matrice et stable dans la matrice, ladite matrice étant physiologiquement acceptable et à base d'hydrocolloïdes physiologiquement compatibles, contenant de 0% à 98% d'humidité, et ladite matrice étant formée d'un réseau tridimensionel délimitant des pores dont la taille est comprise de 2 nm à 300 µm, et ne relargant l'hémoglobine contenue dans la susdite matrice qu'en proportion inférieure à 10%.

Le pansement nécessite la présence d'humidité pour fonctionner, cependant un pansement contenant très peu d'humidité (moins de 5% d'eau) est intéressant car il permet d'augmenter la durée de stockage.

Le pansement défini ci-dessus peut être sous la forme liquide ou sèche.

Selon un mode de réalisation avantageux, l'invention concerne un pansement comprenant une hémoglobine immobilisée dans une matrice, tel que défini ci-dessus, dans lequel ladite matrice est formée d'un réseau tridimensionnel délimitant des pores dont la taille est comprise préférentiellement d'environ 2nm à environ 10µm, préférentiellement d'environ 2nm à environ 1µm, encore plus préférentiellement d'environ 5nm à environ 200nm et préférentiellement d'environ 15nm.

Le pansement défini ci-dessus peut être sous la forme liquide ou sèche.

Dans un autre mode de réalisation, l'invention concerne un pansement comprenant une hémoglobine immobilisée dans une matrice, tel que défini ci-dessus, dans lequel la quantité d'hémoglobine par rapport au poids sec total d'hémoglobine et de matrice est comprise d'environ 0,1% (p/p) à environ 60% (p/p), préférentiellement d'environ 10% (p/p) à environ 50% (p/p), préférentiellement d'environ 15% (p/p) à environ 45% (p/p), préférentiellement d'environ 30% (p/p) à environ 40% (p/p) et plus préférentiellement environ 40% (p/p).

La quantité d'hémoglobine indiquée ici correspond au poids sec d'hémoglobine.

Le pansement défini ci-dessus peut être sous la forme liquide ou sèche.

Selon un mode de réalisation avantageux, l'invention concerne un pansement comprenant une hémoglobine immobilisée dans une matrice, tel que défini ci-dessus, dans lequel le pourcentage d'humidité est supérieur à environ 50% et restant compris d'environ 50% à environ 98%, préférentiellement environ 95%.

Le pansement nécessite la présence d'humidité pour fonctionner, sinon le gradient d'oxygène ne peut se créer.

Le pansement défini ci-dessus sera désigné pansement forme liquide.

Dans un autre mode de réalisation, l'invention concerne un pansement comprenant une hémoglobine immobilisée dans une matrice, tel que défini ci-dessus, dans lequel le pourcentage d'humidité est inférieur à environ 5% et restant compris d'environ 5% à environ 0%, préférentiellement environ 2%.

Le pansement avantageusement ne contient pas d'eau ou une très faible quantité (inférieure à 5% d'eau), permettant ainsi un stockage prolongé de celui-ci et donc une durée de conservation allongée par rapport à la forme humide. Pour que le pansement puisse être fonctionnel, il suffit de le réhydrater.

Le pansement défini ci-dessus sera désigné pansement forme sèche.

Selon un mode de réalisation avantageux, l'invention concerne un pansement comprenant une hémoglobine immobilisée dans une matrice, tel que défini ci-dessus, dans lequel l'hémoglobine est de l'hémoglobine humaine ou d'animal vertébré, modifiée chimiquement, ou réticulée.

Le pansement défini ci-dessus peut être sous la forme liquide ou sèche.

Selon un autre mode de réalisation, l'invention concerne un pansement comprenant une hémoglobine immobilisée dans une matrice, tel que défini ci-dessus, dans lequel l'hémoglobine est une hémoglobine extracellulaire d'animal invertébré, choisie parmi le phylum des annélides et notamment une hémoglobine extracellulaire appartenant à des vers marins tel qu'*Arenicola marina.*

Le pansement défini ci-dessus peut être sous la forme liquide ou sèche.

Dans un autre mode de réalisation, l'invention concerne un pansement comprenant une hémoglobine immobilisée dans une matrice, tel que défini ci-dessus, dans lequel ladite matrice est à base de chitosane, de carraghénanes, de carboxyméthylcellulose, d'alginates et en particulier, d'alginate de sodium ou de calcium.

L'alginate de calcium peut être obtenue par échange d'ion de l'alginate de sodium, par réaction de l'alginate de sodium avec un cation divalent tel que le chlorure de calcium, l'acétate de calcium, le carbonate de calcium, le phosphate de calcium, de préférence le chlorure de calcium.

Le pansement défini ci-dessus peut être sous la forme liquide ou sèche.

Selon un mode de réalisation avantageux, l'invention concerne un pansement comprenant une hémoglobine immobilisée dans une matrice, tel que défini ci-dessus, dans lequel ledit alginate par rapport au poids sec total d'hémoglobine et de matrice est présent à raison d'environ 40%(p/p) à environ 99%(p/p), préférentiellement d'environ 50%(p/p) à environ 90%(p/p), préférentiellement d'environ 55%(p/p) à environ 85%(p/p), préférentiellement d'environ 60%(p/p) à environ 80%(p/p), et préférentiellement environ 60%(p/p).

Le pansement défini ci-dessus peut être sous la forme liquide ou sèche.

Selon un mode de réalisation avantageux, l'invention concerne un pansement comprenant une hémoglobine immobilisée dans une matrice, tel que défini ci-dessus, qui permet la création d'un gradient d'oxygène au travers du gel, de la zone oxygénée vers la zone hypoxique.

La zone oxygénée est la partie externe du pansement en contact avec l'air ambiant, partie qui va capter l'oxygène de l'air.

La zone hypoxique est la partie en contact avec la plaie et qui est déficiente en oxygène.

Dans un autre mode de réalisation, l'invention concerne un pansement comprenant une hémoglobine immobilisée dans une matrice, tel que défini ci-dessus, dans laquelle l'hémoglobine est en association avec un ou plusieurs éléments choisis parmi les cofacteurs, les conservateurs tels que le 4-hydroxybenzoate de méthyle, le 4-hydroxy benzoate de propyle, les antioxydants tels que le glutathion réduit, l'acide ascorbique, le NADH, de la myoglobine humaine ou d'origine animale, ou une combinaison de ces éléments.

Le pansement défini ci-dessus peut être sous la forme liquide ou sèche.

Selon un mode de réalisation avantageux, l'invention concerne un pansement comprenant une hémoglobine immobilisée dans une matrice, tel que défini ci-dessus, possédant un effet bactéricide sur les bactéries anaérobies de type Gram.

Le pansement défini ci-dessus peut être sous la forme liquide ou sèche.

Selon encore un autre aspect, l'invention concerne une composition intermédiaire se présentant sous forme aqueuse comprenant un mélange d'une hémoglobine humaine ou d'animal vertébré ou invertébré et d'un hydrocolloïde non polymérisé, lequel est physiologiquement acceptable, en solution, notamment dans l'eau, ou dans un tampon physiologiquement compatible.

La .composition intermédiaire définie ci-dessus sera désignée composition intermédiaire liquide.

L'hydrocolloïde est non polymérisé, c'est-à-dire que c'est un agent de fixation d'origine végétale composé de polysaccharides et capable de former un gel et choisi parmi le chitosane, les carraghénanes, la carboxyméthylcellulose ou les alginates, tels que les alginates de potassium, lithium, magnésium, ammonium et préférentiellement de sodium.

Selon un autre aspect, l'invention concerne une composition intermédiaire se présentant sous forme séchée contenant moins de 5% d'humidité, comprenant un mélange d'une hémoglobine humaine ou d'animal vertébré ou invertébré et d'un hydrocolloïde physiologiquement acceptable.

La composition intermédiaire définie ci-dessus sera désignée composition intermédiaire sèche.

L'hydrocolloïde est non polymérisé, tel que défini ci-dessus.

Selon un mode de réalisation avantageux, l'invention concerne une composition intermédiaire telle que définie ci-dessus, dans laquelle la quantité d'hémoglobine par rapport au poids total d'hémoglobine et d'hydrocolloïde est comprise d'environ 0,1% (p/p) à environ 60% (p/p), préférentiellement d'environ 10% (p/p) à environ 50% (p/p), préférentiellement d'environ 15% (p/p) à environ 45% (p/p), préférentiellement d'environ 30% (p/p) à environ 40% (p/p) et plus préférentiellement environ 40% (p/p).

La quantité d'hémoglobine indiquée ici est en poids sec.

La composition intermédiaire définie ci-dessus peut être sous la forme liquide ou sèche.

Selon un mode de réalisation avantageux, l'invention concerne une composition intermédiaire telle que définie ci-dessus, dans laquelle l'hémoglobine est de l'hémoglobine humaine ou d'animal vertébré modifiée chimiquement, ou réticulée.

La composition intermédiaire définie ci-dessus peut être sous la forme liquide ou sèche.

Dans un autre mode de réalisation, l'invention concerne une composition intermédiaire telle que définie ci-dessus, dans laquelle l'hémoglobine est une hémoglobine extracellulaire d'animal invertébré, choisie parmi le phylum des annélides et notamment une hémoglobine extracellulaire appartenant à des vers marins tels *qu'Arenicola marina.*

La composition intermédiaire définie ci-dessus peut être sous la forme liquide ou sèche.

Selon un mode de réalisation avantageux, l'invention concerne une composition intermédiaire telle que définie ci-dessus, dans laquelle l'hydrocolloïde est à base de chitosane, de carraghénanes, de carboxyméthylcellulose, d'alginates et en particulier, d'alginate de sodium.

La composition intermédiaire définie ci-dessus peut être sous la forme liquide ou sèche.

Dans un autre mode de réalisation, l'invention concerne une composition intermédiaire telle que définie ci-dessus,dans laquelle ledit alginate par rapport au poids total d'hémoglobine et d'hydrocolloïdes est présent à raison d'environ 40%(p/p) à environ 99%(p/p), préférentiellement d'environ 50%(p/p) à environ 90%(p/p), préférentiellement d'environ 55%(p/p) à environ 85%(p/p), préférentiellement d'environ 60%(p/p) à environ 80%(p/p), et préférentiellement environ 60%(p/p).

La composition intermédiaire définie ci-dessus peut être sous la forme liquide ou sèche.

La quantité d'alginate indiquée ici est en poids sec.

Selon un mode de réalisation avantageux, l'invention concerne une composition intermédiaire telle que définie ci-dessus, dans laquelle l'hémoglobine est en association avec un ou plusieurs éléments choisis parmi les cofacteurs, les conservateurs tels que le 4-hydroxybenzoate de méthyle, le 4-hydroxy benzoate de propyle, les antioxydants tels que le gluthation réduit, l'acide ascorbique, le NADH, de la myoglobine humaine ou d'origine animale, ou une combinaison de ces éléments.

La composition intermédiaire définie ci-dessus peut être sous la forme liquide ou sèche.

Selon encore un autre aspect, l'invention concerne une composition pharmaceutique comprenant comme substance active une hémoglobine, humaine ou d'animal vertébré ou invertébré, essentiellement immobilisée dans une matrice et stable dans la matrice, ladite matrice étant physiologiquement acceptable, en association avec un véhicule pharmaceutiquement acceptable.

Selon un mode de réalisation avantageux, l'invention concerne une composition pharmaceutique telle que définie ci-dessus, se présentant sous forme administrable par voie topique à raison de 0,012 mg/j à 100 mg/j de substance active, préférentiellement de 0,12 mg/j à 100 mg/j et plus préférentiellement de 10 mg/j à 20 mg/j ou sous forme administrable par voie orale à raison de 0,012 mg/kg/j à 100 mg/kg/j de substance active, préférentiellement de 0,12 mg/kg/j à 100 mg/kg/j et plus préférentiellement de 10 mg/kg/j à 20 mg/kg/j.

Les compositions pharmaceutiques administrables par voie topique sont destinées au traitement des maladies parodontales qui concernent tous les tissus de soutien des dents: la gencive (figure 2), le ligament et l'os alvéolaire et celles administrables par voie orale sont destinées à la protection de la muqueuse gastrique en tant que protecteur gastrique pour le traitement notamment des gastrites, des brûlures oesophagiennes ou du météorisme.

Selon encore un autre aspect, l'invention concerne une composition cosmétique comprenant une hémoglobine humaine ou d'animal vertébré ou invertébré essentiellement immobilisée dans une matrice et stable dans la matrice, ladite matrice étant physiologiquement acceptable, en association avec un véhicule cosmétiquement acceptable.

Selon un mode de réalisation avantageux, l'invention concerne une composition cosmétique telle que définie ci-dessus, se présentant sous forme administrable par voie topique à raison de 0,012 mg/j à 100 mg/j de substance active, préférentiellement de 0,12 mg/j à 100 mg/j et plus préférentiellement de 10 mg/j à 20 mg/j.

Les compositions cosmétiques sont destinées à la régénération de la peau dans le cas de déficience en oxygène ou à la prévention de cette déficience et notamment dans le cadre des modifications dégénératives de la peau, de modifications induites par les radiations, par la chaleur ou par l'âge, et particulièrement les rides.

Selon encore un autre aspect, l'invention concerne un procédé de préparation d'un pansement constitué d'une hémoglobine humaine ou d'animal vertébré ou invertébré essentiellement immobilisée dans une matrice à base d'hydrocolloïde et contenant de 0% à 98% d'humidité, comprenant une étape de polymérisation dudit hydrocolloïde contenu dans un mélange d'hydrocolloïde et d'une hémoglobine, lequel mélange contient moins de 5% d'humidité, dans une solution aqueuse d'un cation divalent ou trivalent, ou une solution contenant un agent pontant tels que les dialdéhydes.

Le composé obtenu correspond au pansement sous forme humide contenant de 50 à 98% d'eau.

Compte tenu de l'instabilité de l'hémoglobine, il n'était pas évident pour l'homme de l'art que la polymérisation de l'hydrocolloïde puisse se faire en présence d'hémoglobine et permette d'obtenir comme produit final un pansement conservant toutes ses fonctionnalités.

La solution aqueuse de cation divalent ou trivalent permet d'effectuer la polymérisation de l'hydrocolloïde par échange d'ion de l'hydrocolloïde initial.

Les cations divalents ou trivalents sont choisis parmi le chlorure de calcium, l'acétate de zinc, l'acétate de calcium, le carbonate de calcium, le phosphate de calcium, le chlorure d'aluminium, le glutaraldéhyde, préférentiellement le chlorure de calcium, et utilisés à une concentration de 0,1 à 15%, préférentiellement de 2 à 10% et plus préférentiellement de 10%.

Selon un mode de réalisation avantageux, l'invention concerne un procédé de préparation, tel que défini ci-dessus, dans lequel le mélange d'un hydrocolloïde et d'une hémoglobine contenant moins de 5% d'humidité a été préparé par séchage sous vide d'une solution aqueuse d'un hydrocolloïde et de l'hémoglobine.

Un des avantages du procédé est que la solution aqueuse contenant un hydrocolloïde et une hémoglobine peut être séchée avant polymérisation, contrairement aux techniques habituellement employées, où le séchage intervient après la polymérisation

Le séchage avant polymérisation permet de limiter le relargage de l'hémoglobine et d'obtenir un pansement d'épaisseur fine.

La dite solution aqueuse a été préparée par mélange d'une solution aqueuse d'un hydrocolloïde à une concentration de 1 à 3% avec une solution aqueuse d'hémoglobine à une concentration de 2 à 10 mg/ml.

Selon un mode de réalisation avantageux, l'invention concerne un procédé de préparation, tel que défini ci-dessus, comprenant une étape ultérieure de déshydratation d'un pansement contenant d'environ 50% d'humidité à 98% d'humidité, pour obtenir un pansement contenant moins de 5% d'humidité.

Un des avantages du procédé conduisant au pansement contenant moins de 5% d'humidité (forme sèche) réside dans le fait que ce pansement peut être stocké durant une période de temps plus élevée que la forme humide contenant de 50 à 98% d'eau (forme humide).

Selon un mode de réalisation avantageux, l'invention concerne un procédé de préparation, tel que défini ci-dessus, comprenant une étape ultérieure de réhydratation du pansement contenant moins de 5% d'humidité, pour obtenir un pansement contenant d'environ 40% d'humidité à 70% d'humidité.

Le pansement contenant moins de 5% d'humidité (forme sèche), produit de stockage, peut être avantageusement ultérieurement réhydratée pour être utilisé de la même manière que le pansement humide (forme humide) obtenu directement.

Selon un mode de réalisation avantageux, l'invention concerne un procédé de préparation, tel que défini ci-dessus, dans lequel hémoglobine extracellulaire d'animal invertébré, choisie parmi le phylum des annélides et notamment une hémoglobine extracellulaire appartenant à des vers marins tels qu'*Arenicola marina.*

Selon un autre mode de réalisation, l'invention concerne un procédé de préparation, tel que défini ci-dessus, dans lequel l'hydrocolloïde est à base de chitosane, dé carraghénanes, de carboxyméthylcellulose, d'alginates et en particulier, d'alginate de sodium ou de calcium.

Selon un mode de réalisation avantageux, l'invention concerne un procédé de préparation, tel que défini ci-dessus, comprenant les étapes suivantes :
a. brassage d'une solution contenant une hémoglobine et d'une solution contenant un hydrocolloïde, la proportion d'hémoglobine par rapport au poids sec total d'hémoglobine et d'hydrocolloide étant comprise d'environ 0,1% (p/p) à environ 60% (p/p), préférentiellement d'environ 10% (p/p) à environ 50% (p/p), préférentiellement d'environ 15% (p/p) à environ 45% (p/p) et plus préférentiellement environ 40% (p/p), pour obtenir une composition intermédiaire sous forme aqueuse constitué d'un mélange d'hémoglobine et d'un hydrocolloïde en solution,
b. séchage sous vide dudit mélange d'hémoglobine et d'un hydrocolloïde en solution pour obtenir un mélange d'hémoglobine et d'un hydrocolloïde contenant moins de 5% d'humidité,
c. polymérisation dudit hydrocolloïde dans ledit mélange contenant moins de 5% d'humidité dans une solution aqueuse d'un cation divalent choisi parmi le chlorure de calcium, l'acétate de zinc, l'acétate de calcium, le carbonate de calcium, le phosphate de calcium, le chlorure d'aluminium ou des dialdéhydes et préférentiellement le chlorure de calcium à une concentration de 0,1 à 5%, préférentiellement de 2 à 10% et plus préférentiellement de 10%, pour obtenir un pansement contenant de 50% à 98% d'humidité,
d. optionnellement, déshydratation par évaporation sous vide et/ou lyophilisation dudit pansement contenant de 50% à 98% d'humidité, pour obtenir un produit de stockage contenant moins de 5% d'humidité,
e. optionnellement, réhydratation dudit produit de stockage contenant moins de 5% d'humidité pour obtenir un pansement contenant de 40% à 70% d'humidité.

Selon un autre aspect, l'invention concerne un pansement contenant de 0% à 98% d'humidité, tel qu'obtenu par le procédé défini ci-dessus.

Selon un autre aspect, l'invention concerne un produit de combinaison contenant d'une part une composition intermédiaire sous forme aqueuse telle que définie ci-dessus, et d'autre part, une solution aqueuse d'un cation divalent ou trivalent, ou une solution aqueuse contenant un agent pontant tels que les dialdéhydes, pour fabriquer un pansement contenant de 50% à 98% d'humidité, par polymérisation *in situ* dudit hydrocolloïde contenu dans ladite composition intermédiaire dans ladite solution aqueuse.

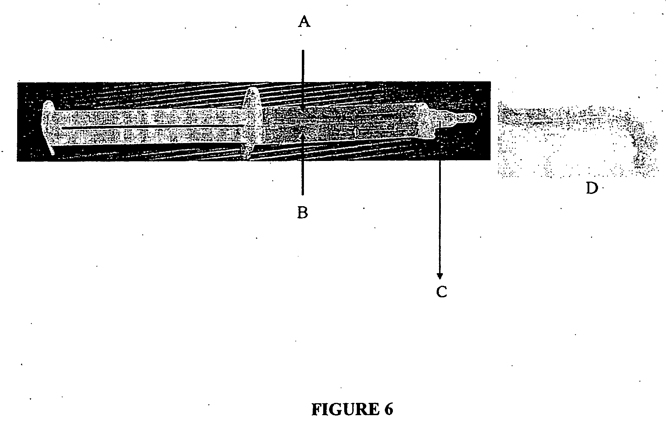

L'un des avantages de l'invention est de fournir un pansement qui peut être fabriqué in situ (figure 6), utile notamment pour le traitement des maladies parodontales, le pansement pouvant être appliqué directement dans la cavité buccale, ou pour le traitement des plaies profondes.

### DESCRIPTION DES FIGURES

La figure 1 représente un exemple de plaie chronique : patient diabétique souffrant d'ischémie au niveau du pied.
La figure 2 représente une atteinte du parodonte par des pathogènes anaérobies, créant des poches entre la gencive et l'organe dentaire.
La figure 3 représente la photographie de la microscopie à balayage (JEOL JEM 1200EX) effectuée sur la face externe de la matrice d'alginate de calcium contenant de l'hémoglobine *d'Arenicola marina* après ombrage à l'or. La résolution utilisée ici ne permet pas de visualiser l'hémoglobine.
La figure 4 représente la photographie de la microscopie à balayage effectuée sur la face interne de la matrice d'alginate de calcium contenant de l'hémoglobine d'*Arenicola marina* (6 mg/ml) après ombrage à l'or et montrant l'aspect poreux de la structure. La résolution utilisée ici ne permet pas de visualiser l'hémoglobine.
La figure 5 montre une représentation de Barcroft (Hémoglobine d'*Arenicola marina* 10 mg/ml, Alginate de sodium1 %, Ca 15 %, Tampon Hepes pH 7.35) permettant de déterminer la P₅₀.
La figure 6 représente une photo de la seringue permettant de fabriquer un pansement in situ. La seringue est constituée de deux compartiments contenant d'une part l'alginate de sodium (Satialgine™ US 61) en solution aqueuse avec l'hémoglobine et d'autre part une solution aqueuse de chlorure de calcium permettant la polymérisation de l'alginate in situ, en sortie de seringue.
La figure 7 représente la polymérisation par dialyse sur support Minicell « Millipore. ».
La figure 8 représente la polymérisation en chambre de diffusion.
La figure 9 représente le relargage d'hémoglobine d'*Arenicola marina* (HbA) contenue dans un pansement préparé avec de l'alginate Satialgine™ US551 (Cargill) à 1% en fonction du temps.
Les figures 10, 11, 12 représentent les chromatogrammes HPLC à deux longueurs d'onde (280 et 414 nm) obtenus sur des prélèvements effectués respectivement à 1h30, 8h et 24h, dans une solution iso-ionique au sang humain (voir exemple 5) dans laquelle un pansement contenant de l'hémoglobine d'*Arenicola marina* a été placé.
La figure 13 représente le chromatogramme HPLC témoin aux deux longueurs d'onde (280 et 414 nm), obtenu avec un pansement sans hémoglobine à 24h.

### PARTIE EXPERIMENTALE

### Exemple 1 : Purification de l'hémoglobine d'Arenicola marina.

Les vers proviennent d'une ferme d'élevage SeaBait. Ces vers sont congelés à -80°C ce qui provoque un choc hémorragique et un éclatement de la paroi du vers ; l'extraction de l'hémoglobine est ainsi facilitée.

Les vers une fois décongelés pendant 24h à 4°C en présence du tampon d'extraction (400mM NaCl, 2.95 mM KCl, 32 mM MgSO4, 11mM CaCl2, 50 mM Hepes, 5mM Acide ascorbique, 10mM glutathion réduit, pH 7.5, filtré 0.2µm) à raison de 0.2 ml/g, sont centrifugés (4500g, 4°C, 15min). Le surnageant est récupéré et le culot de vers est redispersé dans 0.2ml/g de tampon d'extraction et centrifugé à nouveau, et ceci 4 fois. La communelle des surnageants est filtrée sous pression (2 bars) sur filtre 5µm puis 0.1µm (filtres PALL).

Le filtrat peut-être traité de deux façons :
- Première méthode : le filtrat est diafiltré contre 5 diavolumes de tampon de stockage (90 mM naCl, 23 mM gluconate de sodium, 27 mM acétate de sodium, 5 mM KCl, 1.5. mM MgCl2, 2.5 mM CaC12, pH 7.35, filtré 0.2µm) sur cassette d'ultrafiltration Pellicon XL-1000 kDa (Millipore), à 4°C. Le rétentat est finalement concentré sur cette même cassette à ∼100mg/mL avant filtration stérilisante sur filtre 0.2µm et stockage à -80°C.
- Deuxième méthode : le filtrat de 5µm est précipité au point isoélectrique de l'hémoglobine *d'Arenicola marina* en ajoutant 50% en volume d'une solution de 0.5N acétate de sodium/acide acétique, pH 4.15. Après 30 minutes d'agitation à 4°C, la solution est centrifugée (4500g, 4°C, 15min). Le surnageant est éliminé et le culot (contenant l'hémoglobine d'*Arenicola marina*) est lavé 2 fois contre le même équivalent volume d'eau ultrapure (4500g, 4°C, 5 min). Le culot rincé est redispersé dans le même équivalent volume du tampon de stockage sous agitation pendant 1h à 4°C. La solution esr centrifugée pour éliminer les débris (4500g, 4°C, 15 min). Le surnageant est filtré sous pression sur filtre 0.1µm (PALL) puis diafiltré contre 2 diavolumes sur cassette Pellicon-XL-1000kDa à une concentration finale de ∼100mg/mL avant filtration stérilisante sur filtre 0.2µm et stockage à -80°C.

### Exemple 2 : Préparation de la composition intermédiaire liquide

Deux types d'Alginate de sodium commercialisés par Cargill : Satialgine^{™} US 61 et Satialgine^{™} US 551 EP ont été utilisés. Ils sont conformes aux normes de pharmacopée européenne et utilisés comme additif en tant que texturant : épaississant et/ou gélifiant pour de nombreuses applications thérapeutiques.

Ils sont utilisés à une concentration de 1 à 3% (p/v) selon le type d'application et la texture finale souhaitée. La poudre d'alginate de sodium est diluée sous agitation magnétique dans de l'eau MilliQ (MQ) à la concentration souhaitée. Plus la concentration en alginate est élevée plus la dissolution est difficile.

D'autre part, le Satialgine^{™} US 551 EP est plus visqueux que le Satialgine^{™} US 61. Il est donc parfois nécessaire de chauffer (∼50°C) pour améliorer la dissolution. Une fois que la solution est homogène, après quelques heures, elle est refroidie dans de la glace avant d'y ajouter l'hémoglobine d'*Arenicola marina.*

L'hémoglobine d'*Arenicola marina* est préparée comme décrit précédemment et stockée à -80°C avant utilisation, à une concentration de 100mg/mL dans un tampon physiologique dit tampon de stockage dépourvu de calcium.

L'hémoglobine d'*Arenicola marina* est décongelée à 4°C et dissoute dans la solution d'alginate de sodium à la concentration de 6 mg/mL. La solution est ensuite homogénéisée sous agitation magnétique.

### Exemple 3 : Préparation de la composition intermédiaire séchée

La solution obtenue dans l'exemple 2 est ensuite séchée sous vide d'air et en présence de gel de silice entre 12 et 24h pour obtenir une composition intermédiaire séchée.

### Exemple 4 : Polymérisation

La composition intermédiaire séchée qui se présente sous forme d'un film fin, est trempée dans 10mL d'une solution de chlorure de calcium à 1% (p/v). La solution de calcium est tamponnée avec 10mM Hepes (Sigma) à pH 7.0. Cette étape permet la polymérisation de la solution d'alginate et l'immobilisation de l'hémoglobine d'*Arenicola marina* dans la matrice d'alginate.

Plusieurs techniques ont été utilisées pour polymériser la solution d'alginate de sodium contenant l'hémoglobine. Selon le procédé utilisé, il est possible d'obtenir différentes formes de pansement et donc d'envisager le traitement de différent type de plaies ou d'infections parodontales lié à la présence de pathogènes anaérobies.

### 4.1 : Polymérisation in situ

Des seringues doubles (Plas-pak) sont utilisées pour cette application. Un compartiment de la seringue (A) est rempli avec une solution contenant de l'alginate de sodium (Satialgine™ US 61) à 1 % et de l'hémoglobine à 5mg/ml.

L'autre compartiment (B) est rempli avec une solution de CaC12 à 1 % dans un tampon Hepes 10mM, pH7.0.

Une pression exercée sur le piston permet de mettre en contact les solutions des deux compartiments au niveau de l'extrémité (C) de la seringue et de polymériser l'alginate en solution contenant l'hémoglobine (D).

### 4.2 : Polymérisation par dialyse

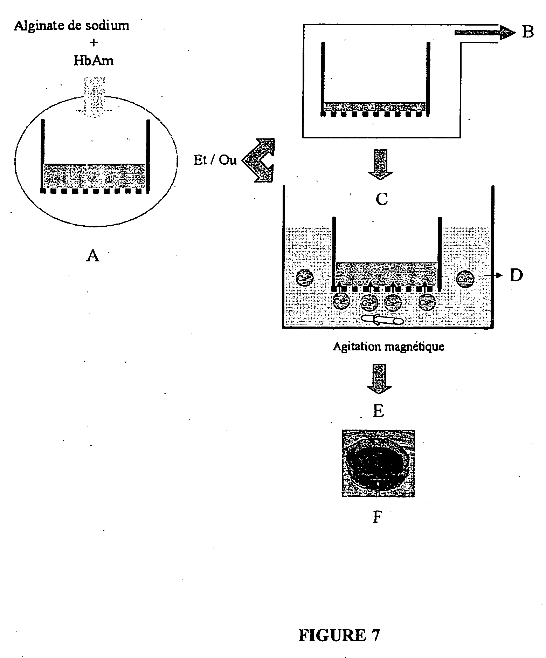

Le principe de la polymérisation est détaillé sur la figure 7.

La solution d'alginate de sodium contenant l'hémoglobine est déposée sur une membrane poreuse (A) (Minicell, 0.4µm, Millipore). (HbAm = Hémoglobine d'*Arenicola marina*).

La solution peut être dégazée et séchée sous vide et en présence de gel de silice (B), avant polymérisation, entre 12h et 24h, ou polymérisée en l'état.

La polymérisation a lieu en trempant la membrane poreuse (C) dans un bain contenant une solution à 1% CaCl₂, 10mM Hepes, pH 7.0 et sous agitation (D). Le calcium diffuse au travers de la membrane pendant les 12h. La polymérisation a lieu à 4°C.

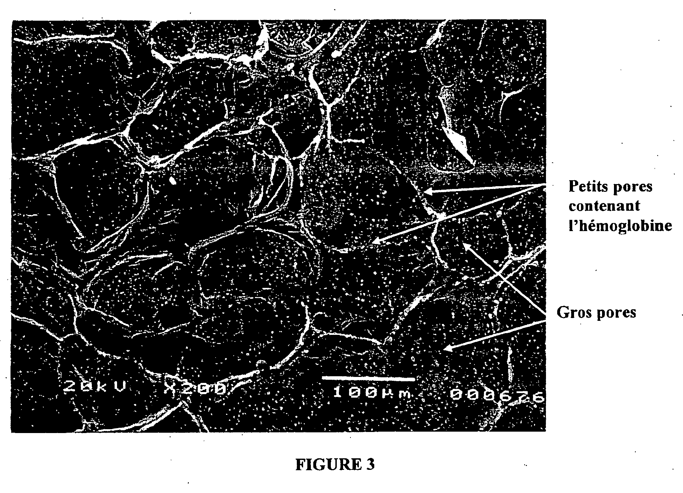

Le polymère obtenu est ensuite rincé abondamment contre H₂O MilliQ (E) puis stocké à 4°C (F). Le gel obtenu a été analysé par microscopie à balayage (Figure 3 et 4).

Il est possible de sécher le gel sous vide par évaporation ou par lyophilisation pour le conserver et le réhydrater avant utilisation (F).

### 4.3 : Polymérisation par chambre de diffusion

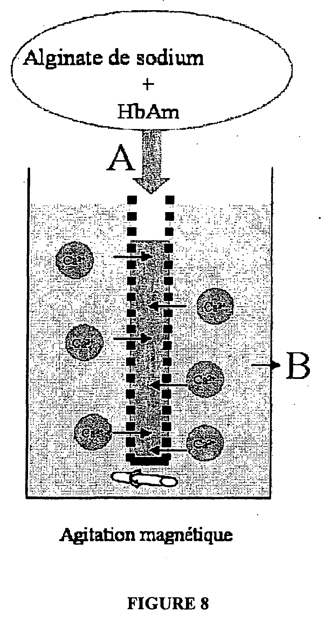

La polymérisation (figure 8) a lieu de part et d'autre de la solution aqueuse d'alginate de sodium (1%) et d'hémoglobine d'*Arenicola marina* (A) qui est maintenue entre deux membranes poreuses (1µm) dans un bain contenant une solution à 1% CaCl₂, 10mM Hepes, pH 7.0 et sous agitation (B).

### Exemple 5 : Relargage de l'hémoglobine

Le pansement, préparé selon l'une des méthodes décrites précédemment, est trempé dans 10ml d'une solution iso-ionique au sang humain (145mM NaCl, 4mM KCl, 2mM MgCl2, 10 mM Hepes, 2.5 mM CaCl2, pH 7.35) et le tout est incubé à 34°C afin de simuler le milieu physiologique de la plaie.

A intervalle de temps régulier, une quantité de solution (0,5 ml) est prélevée pour dosage de l'hémoglobine relarguée avec le réactif de Drabkin avec lecture au spectrophotomètre à 540 nm (dosage colorimétrique). Les concentrations ainsi obtenues dans le volume de 0,5 ml sont converties en quantité totale d'hémoglobine relarguée par le pansement.

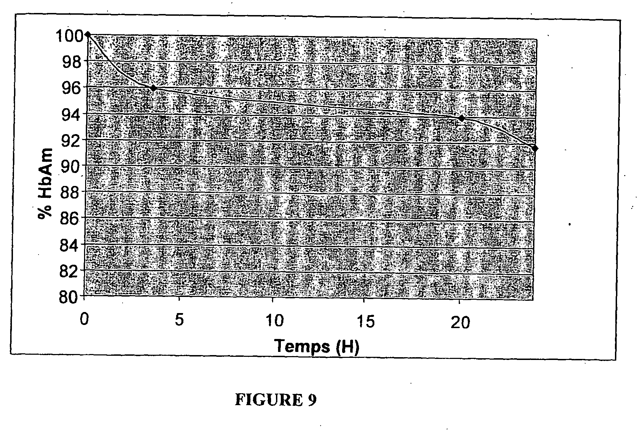

La figure 9 présente les résultats obtenus :
- Après 4h en solution, le pansement n'a rélarguée que 4 % d'hémoglobine.
- Après 20h, le pansement n'a relarguée que 6 % d'hémoglobine.
- Après 24h, seulement 8% de l'hémoglobine contenue dans le pansement a été relarguée.

Ces trois mesures confirment que l'hémoglobine reste immobilisée dans la matrice durant plus de 24 heures.

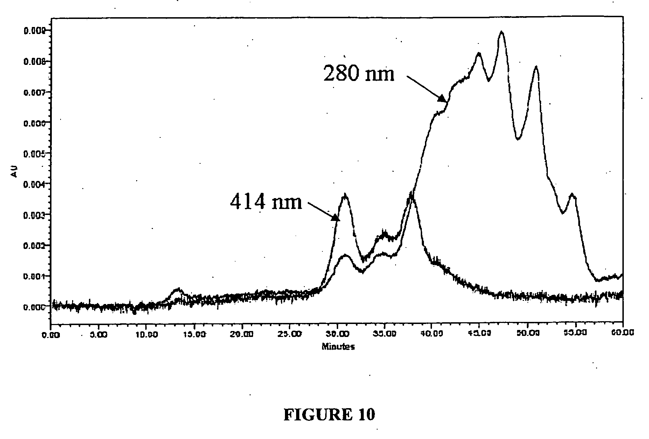

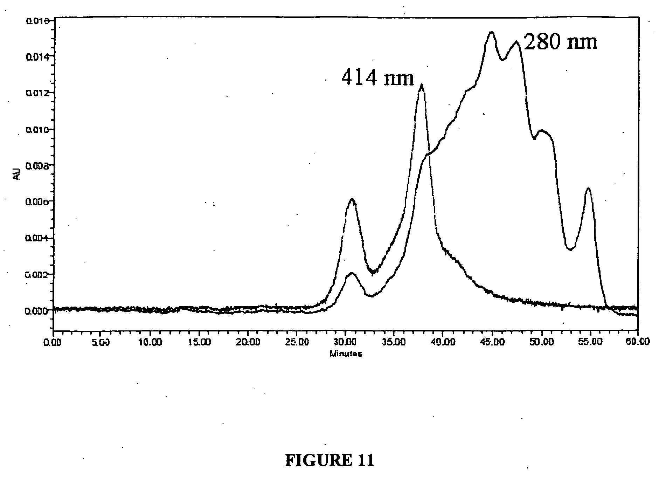

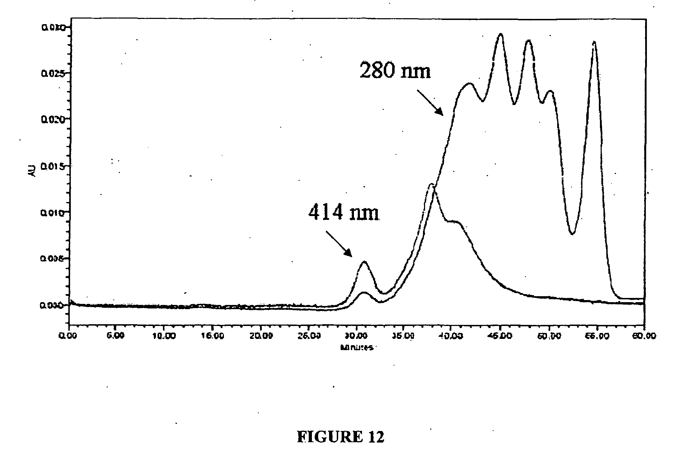

Une analyse de la structure de l'hémoglobine relarguée par chromatographie d'exclusion stérique (colonne superose 6, 0.5 mL/min) a été réalisée. Des prélèvements ont été effectués à différents temps (1h30, 8h et 24 h) et la densité optique a été mesurée à 280 nm et 414 nm (Figure 10, 11, 12).

Les figures 10, 11 et 12 présentent les chromatogrammes obtenus et montrent que l'hémoglobine relarguée est une hémoglobine dégradée (sous forme de dodécamère, pic à 30 minutes), car le pic d'absorption de l'hémoglobine native qui se situe à 21 minutes est inexistant.

Par conséquent, le faible pourcentage d'hémoglobine qui se relargue (moins de 10%) est de l'hémoglobine dégradée, confirmant ainsi que l'hémoglobine d'*Arenicola marina* restant dans le pansement est stable (sinon elle serait relarguée).

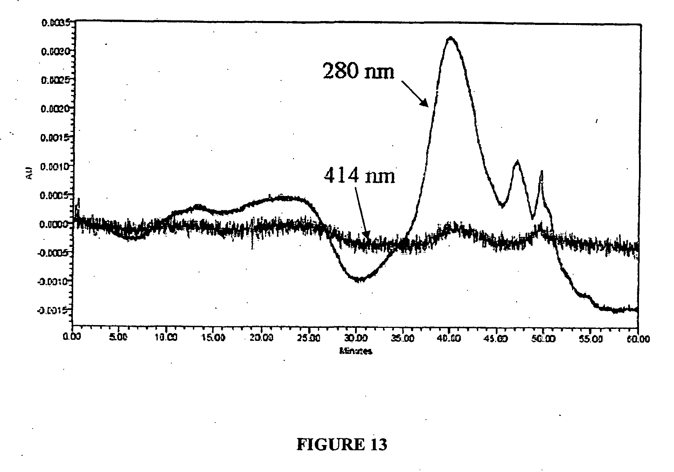

La figure 13 présente le chromatogramme obtenu avec un pansement « contrôle» sans hémoglobine, présentant ainsi l'absorption due uniquement au pansement.

### Exemple 6 : Comparaison avec la préparation de la demande US2003/0180365

La préparation de la demande US2003/0180365 a été préparée comme décrit en y insérant 6mg/mL d'hémoglobine d'*Arenicola marina.*

Les tests de relargage de l'hémoglobine d'*Arenicola marina* ont été réalisés comme décrit dans l'exemple 5.

La formulation ainsi obtenue a l'aspect d'un gel très visqueux et une fois trempée dans la solution iso-ionique au sang humain comme décrit dans l'exemple 5, on observe une liquéfaction complète du gel au bout de 12h et donc un relargage total de l'hémoglobine d'*Arenicola marina.*

## Revendications

1. Utilisation d'une hémoglobine, humaine ou d'animal vertébré ou invertébré, immobilisée dans une matrice à base d'hydrocolloïdes polymérisés et stable dans la matrice, ladite matrice étant physiologiquement acceptable, et contenant de 0 à 98% d'humidité, et ladite matrice étant formée d'un réseau tridimensionnel délimitant des pores dont la taille est comprise de 2nm à 300µm,
comme transporteur d'oxygène dans un tissu physiologique nécessitant un apport d'oxygène, pour la préparation d'un pansement destiné au traitement externe de plaies ouvertes, profondes ou chroniques, ou de maladies parodontales, ou d'un pansement gastrique ou d'un pansement utilisable pour les tissus, sans que le relargage de l'hémoglobine immobilisée dans la susdite matrice ne soit supérieur à 10%.

2. Utilisation d'une hémoglobine immobilisée dans une matrice selon la revendication 1, dans laquelle la quantité d'hémoglobine par rapport au poids sec total d'hémoglobine et de matrice est comprise de 0,1 % (p/p) à 60% (p/p).

3. Utilisation d'une hémoglobine immobilisée dans une matrice selon l'une des revendications 1 ou 2, dans laquelle l'hémoglobine est de l'hémoglobine humaine ou d'animal vertébré, modifiée chimiquement, ou réticulée.

4. Utilisation d'une hémoglobine immobilisée dans une matrice selon l'une des revendications 1 ou 2, dans laquelle l'hémoglobine est une hémoglobine extracellulaire d'animal invertébré, choisie parmi le phylum des annélides.

5. Utilisation d'une hémoglobine immobilisée dans une matrice selon la revendication 4, dans laquelle l'hémoglobine est une hémoglobine extracellulaire appartenant à Arenicola marina.

6. Utilisation d'une hémoglobine immobilisée dans une matrice selon l'une des revendications 1 à 5, dans laquelle ladite matrice est à base de chitosane, de carraghénanes, de carboxyméthylcellulose, d'alginates.

7. Utilisation d'une hémoglobine immobilisée dans une matrice selon l'une des revendications 1 à 6, dans laquelle ladite matrice est à base d'alginate de calcium.

8. Utilisation d'une hémoglobine immobilisée dans une matrice selon la revendication 6 ou 7, dans laquelle ledit alginate par rapport au poids total d'hémoglobine et de matrice est présent à raison de 40%(p/p) à 99%(p/p).

9. Pansement comprenant une hémoglobine, humaine ou d'animal vertébré ou invertébré, immobilisée dans une matrice et stable dans la matrice, ladite matrice étant physiologiquement acceptable et à base d'hydrocolloïdes physiologiquement compatibles, contenant de 0% à 98% d'humidité, et ladite matrice étant formée d'un réseau tridimensionnel délimitant des pores dont la taille est comprise de 2nm à 300µm, et ne relarguant l'hémoglobine contenue dans la susdite matrice qu'en proportion inférieure à 10%.

10. Composition intermédiaire se présentant sous forme séchée contenant moins de 5% d'humidité, comprenant un mélange d'une hémoglobine humaine ou d'animal vertébré ou invertébré et d'un hydrocolloïde physiologiquement acceptable.

11. Composition pharmaceutique comprenant comme substance active une hémoglobine, humaine ou d'animal vertébré ou invertébré, immobilisée dans une matrice et stable dans la matrice, ladite matrice étant formée d'un réseau tridimensionnel délimitant des pores dont la taille est comprise de 2nm à 300µm, et ladite matrice étant physiologiquement acceptable.

12. Composition cosmétique comprenant une hémoglobine humaine ou d'animal vertébré ou invertébré immobilisée dans une matrice et stable dans la matrice, ladite matrice étant formée d'un réseau tridimensionnel délimitant des pores dont la taille est comprise de 2nm à 300µm, et ladite matrice étant physiologiquement acceptable.

13. Procédé de préparation d'un pansement selon la revendication 9, constitué d'une hémoglobine humaine ou d'animal vertébré ou invertébré immobilisée dans une matrice à base d'hydrocolloïde et contenant de 0% à 98% d'humidité, comprenant une étape de polymérisation dudit hydrocolloïde contenu dans un mélange d'hydrocolloïde et d'une hémoglobine, ledit mélange constituant ladite composition intermédiaire définie dans la revendication 10, dans laquelle l'hémoglobine est une hémoglobine extracellulaire d'animal invertébré, choisie parmi le phylum des annélides,
et lequel mélange contient moins de 5% d'humidité, dans une solution aqueuse d'un cation divalent ou trivalent, ou une solution contenant un agent pontant tels que les dialdéhydes.

14. Procédé de préparation d'un pansement selon la revendication 13, dans lequel le mélange d'un hydrocolloïde et d'une hémoglobine contenant moins de 5% d'humidité a été préparé par séchage sous vide d'une solution aqueuse d'un hydrocolloïde en association avec une hémoglobine.

15. Procédé de préparation d'un pansement selon l'une des revendications 13 ou 14, dans laquelle l'hémoglobine est une hémoglobine extracellulaire appartenant à Arenicola marina.

## Claims

1. Use of a hemoglobin of human or vertebrate or invertebrate animal, immobilized in a matrix made from polymerized hydrocolloids and stable in the matrix, said matrix being physiologically acceptable, and containing 0-98% humidity, and said matrix being formed of a three-dimensional network delimiting pores whose size ranges from 2 nm to 300µm, as an oxygen-carrier in a physiological tissue requiring oxygen supply, for the preparation of a dressing for the treatment of external open wounds, deep or chronic, or of periodontal diseases, or of a gastric demulsent or of a dressing for tissues, the release of hemoglobin immobilized in the above matrix being not greater than 10%.

2. Use of a hemoglobin immobilized in a matrix according to claim 1, wherein the amount of hemoglobin relative to total dry weight of hemoglobin and matrix is comprised between 0.1% (w / w) and 60% (w / w).

3. Use of a hemoglobin immobilized in a matrix according to any one of claims 1 or 2, wherein hemoglobin is human or vertebrate animal hemoglobin, chemically modified, or crosslinked.

4. Use of a hemoglobin immobilized in a matrix according to any one of claims 1 or 2, wherein hemoglobin is an extracellular hemoglobin of invertebrate animals, selected from the phylum of Annelids.

5. Use of a hemoglobin immobilized in a matrix according to claim 4, wherein hemoglobin is an extracellular hemoglobin of Arenicola marina.

6. Use of a hemoglobin immobilized in a matrix according to any one of claims 1 to 5, wherein said matrix is made from chitosan, carrageenan, carboxymethylcellulose, alginates.

7. Use of a hemoglobin immobilized in a matrix according to any one of claims 1 to 6, wherein said matrix is made from calcium alginate.

8. Use of a hemoglobin immobilized in a matrix according to claim 6 or 7, wherein said alginate compared to the total weight of hemoglobin and matrix is present in 40% (w / w) to 99% (w / w).

9. Dressing comprising a hemoglobin of human or animal vertebrate or invertebrate, immobilized in a matrix and stable in the matrix, said matrix being physiologically acceptable and made from physiologically compatible hydrocolloids, containing 0% to 98% humidity, and said matrix being formed of a three-dimensional network delimiting pores whose size ranges from 2 nm to 300µm, and salting out of the hemoglobin contained in the above matrix only in a proportion below 10%.

10. Intermediate composition in a dried form containing less than 5% humidity, comprising a mixture of hemoglobin of human or of a vertebrate or invertebrate animal and a physiologically acceptable hydrocolloid.

11. Pharmaceutical composition comprising as an active substance a hemoglobin, human or of vertebrate or invertebrate animal, immobilized in a matrix and stable in the matrix, said matrix being made from a three-dimensional network delimiting pores whose size ranges from 2 nm to 300µm, and said matrix being physiologically acceptable.

12. Cosmetic composition comprising a human hemoglobin or of a vertebrate or invertebrate animal immobilized in a matrix and stable in the matrix, said matrix being made from a three-dimensional network delimiting pores whose size ranges from 2 nm to 300µm, and said matrix being physiologically acceptable.

13. A process for preparing a dressing according to claim 9, consisting of a human hemoglobin or of a vertebrate or invertebrate animal immobilized in a matrix made from hydrocolloid and containing 0% to 98% humidity, comprising a step of polymerization of said hydrocolloid contained in a mixture of hydrocolloid and a hemoglobin, said mixture being said intermediate composition defined in claim 10, in which the hemoglobin is an extracellular hemoglobin of invertebrate animal, selected from the phylum of Annelids, and said mixture contains less than 5% humidity, in an aqueous solution of a divalent or trivalent cation, or a solution containing a bridging agent such as dialdehydes.

14. A process for preparing a dressing according to claim 13, wherein the mixture of a hydrocolloid and a hemoglobin containing less than 5% moisture has been prepared by vacuum drying of an aqueous solution of a hydrocolloid in combination with a hemoglobin.

15. A process for preparing a dressing according to any one of claims 13 or 14, wherein the hemoglobin is an extracellular hemoglobin of Arenicola marina.

## Patentansprüche

1. Verwendung von einem humanen, tierisch vertebraten oder invertebraten Hämoglobin, immobilisiert in einer Matrix, worin die Matrix auf polymerisierten Hydrocolloiden basiert und worin das Hämoglobin stabil in der Matrix ist, die Matrix physiologisch akzeptabel ist und 0% bis 98% Feuchtigkeit enthält und die Matrix aus einem dreidimensionalen Netzwerk gebildet ist welches Poren mit einer Größe von 2nm bis 300µm begrenzt, als Sauerstofftransporter in einem physiologischen Gewebe welches einen Sauerstoffträger benötigt, für die Herstellung eines Verbandes, welcher zur externen Behandlung offener, tiefer oder chronischer Wunden, oder parodontaler Erkrankungen bestimmt ist, oder eines gastrischen Verbandes oder eines Verbandes welcher für Gewebe verwendbar ist, ohne das die Freisetzung des in der Matrix immobilisierten Hämoglobins mehr als 10% beträgt.

2. Verwendung eines in einer Matrix immobilisierten Hämoglobins nach Anspruch 1, worin die Menge an Hämoglobin, bezogen auf die Gesamttrockenmasse von Hämoglobin und Matrix 0,1 bis 60 Gew.-% beträgt.

3. Verwendung eines in einer Matrix immobilisierten Hämoglobins nach Anspruch 1 oder 2, worin das Hämoglobin chemisch modifiziertes oder vernetztes humanes oder tierisch vertebrates Hämoglobin ist.

4. Verwendung eines in einer Matrix immobilisierten Hämoglobins nach Anspruch 1 oder 2, worin das Hämoglobin ein extrazelluläres Hämoglobin von Invertebraten, ausgewählt aus dem Stamm Annelida ist.

5. Verwendung eines in einer Matrix immobilisierten Hämoglobins nach Anspruch 4, worin das Hämoglobin ein extrazelluläres Hämoglobin von Arenicola marina ist.

6. Verwendung eines in einer Matrix immobilisierten Hämoglobins nach einem der Ansprüche 1 bis 5, worin die Matrix basiert auf Chitosan, Carrageenan, Carboxymethylcellulose, Alginaten.

7. Verwendung eines in einer Matrix immobilisierten Hämoglobins nach einem der Ansprüche 1 bis 6, worin die Matrix auf Calciumalginat basiert.

8. Verwendung eines in einer Matrix immobilisierten Hämoglobins nach Anspruch 6 oder 7, worin das Alginat, bezogen auf das Gesamtgewicht von Hämoglobin und der Martrix, zu 40 bis 99 Gew.-% enthalten ist.

9. Verband umfassend ein humanes, tierisch vertebrates oder invertebrates Hämoglobin, immobilisierte in einer Matrix und in der Matrix stabil, worin die Matrix physiologisch akzeptabel ist und auf physiologisch kompatiblen Hydrocolloiden basiert, mit einem Feuchtigkeitsgehalt von 0 bis 98% und worin die Matrix aus einem dreidimensionales Netzwerk gebildet ist welches Poren mit einer Größe von 2nm bis 300µm begrenzt und worin die Matrix nicht weniger als 10% des in der Matrix enthaltenen Hämoglobins freisetzt.

10. Eine intermediäre Zusammensetzung, welche in getrockneter Form mit einem Feuchtigkeitsgehalt von weniger als 5% vorliegt, umfassend ein Gemisch aus humanen, tierisch vertebraten oder invertebraten Hämoglobin und einem physiologisch annehmbaren Hydrocolloids.

11. Pharmazeutische Zusammensetzung, umfassend als Wirkstoff humanes, tierisch vertebrates oder invertebrates Hämoglobin, welches in einer Matrix immobilisiert und in der Matrix stabil ist, wobei die Matrix aus einem dreidimensionales Netzwerk gebildet ist welches Poren mit einer Größe von 2nm bis 300µm begrenzt und die Matrix physiologisch akzeptable ist.

12. Kosmetisches Zusammensetzung, umfassend ein humanes, tierisch vertebrates oder invertebrates Hämoglobin, welches in einer Matrix immobilisiert und in der Matrix stabil ist, wobei die Matrix aus einem dreidimensionalen Netzwerk gebildet ist welches Poren mit einer Größe von 2nm bis 300µm begrenzt und die Matrix physiologisch akzeptable ist.

13. Verfahren zur Herstellung eines Verbandes nach Anspruch 9, gebildet aus humanen, tierisch vertebraten oder invertebraten Hämoglobin, welches in einer Matrix auf Basis von Hydrocolloid mit einem Feuchtigkeitsgehalt von 0% bis 98% immobilisiert ist, umfassend einen Polymerisationsschritt des Hydrocolloids in einer Mischung von Hydrocolloid und Hämoglobin, wobei die Mischung die in Anspruch 10 definierte intermediäre Zusammensetzung bildet, worin das Hämoglobin ein extrazelluläres invertebraten Hämoglobin ausgewählt aus dem Stamm Annelida ist, und worin die Mischung weniger als 5% Feuchtigkeit in einer wässrigen Lösung eines divalenten- und trivaltenten-Kations oder einer Lösung welche ein Brückenbildungsmittel wie z.B. Dialdehyde enthält.

14. Verfahren zur Herstellung eines Verbandes nach Anspruch 13, worin die Mischung aus Hydrocolloid und Hämoglobin, die weniger als 5% Feuchtigkeit enthält, durch Vakuum-Trocknung einer wässrigen Lösung eines Hydrocolloids in Kombination mit Hämoglobin hergestellt worden ist.

15. Verfahren zur Herstellung eines Verbandes nach Anspruch 13 oder 14, worin das Hämoglobin ein extrazelluläres Hämoglobin aus Arenicola marina ist.
